# EUROPEAN PATENT APPLICATION

(11) **EP 4 616 886 A1**
(43) Date of publication of application: **17.09.2025**
(21) Application number: 24162983.1
(22) Date of filing: 12.03.2024
(51) Int. Cl.: A61M 5/315, A61M 5/50

(54) **PLUNGER ROD AND MEDICAL CONTAINER INCLUDING THIS PLUNGER ROD**

(71) Applicant: Becton Dickinson Holdings Pte. Ltd., Singapore 639461 (SG)
(72) Inventor: VARGEESE, Raja, 625009 Tamil nadu (IN); SUNDARALINGAM, Mahesh Kumar, Puthalam, Tamilnadu 629602 (IN)
(74) Representative: Germain Maureau

(57) **Abstract**

This plunger rod (1) is configured for pushing a stopper (108) to expel a medical product (107) contained in a medical container (100). The plunger rod (1) includes a first part (20) having a proximal end (201) and a distal end (220) configured to push the stopper (108), and a second part (30) removably attached to the proximal end (201) of the first part (20), the second part (30) including a pushing surface (306) for allowing a user to push the second part (30) together with the first part (20) in the distal direction to perform injection. The second part (30) is configured to detach from the first part (20) if pulled in the proximal direction after injection.

## Description

The present invention relates to a plunger rod and a medical container including this plunger rod.

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction away from the user's hand, and the "proximal direction" is to be understood as meaning the direction toward the user's hand.

Conventional medical containers include a barrel forming a reservoir for containing a medical product, a stopper arranged within the barrel and a plunger rod configured to push the stopper in the distal direction to expel the medical product into an injection site.

Once an injection operation is completed, the medical container is normally thrown away in a sharps container. It may however happen that some users reuse medical containers, first by refilling the barrel and then by operating another injection.

There is therefore a need for a device that would prevent reuse of a medical container once an injection has been performed.

In this context, an aspect of the invention is a plunger rod configured for pushing a stopper to expel a medical product contained in a medical container, the plunger rod including
a first part having a proximal end and a distal end configured to push the stopper,
a second part removably attached to the proximal end of the first part, the second part including a pushing surface for allowing a user to push the second part together with the first part in the distal direction to perform injection, wherein the second part is configured to detach from the first part if pulled in the proximal direction after injection.

As a result, when a user attempts to extract drug from a vial by means of a medical container comprising the plunger rod of the invention, and by pulling this plunger rod in the proximal direction, the second part will get detached from the first part of the plunger rod. The stopper and the first part of the plunger rod will thus remain inside the syringe barrel. Thus, refilling and reusing are prevented.

The plunger rod includes securing means for securing the second part to the first part. The securing means are configured to maintain the first part and the second part assembled to each other during storage and injection, i.e. during distal movement of the plunger rod with respect to the medical container. However, the securing means are also configured to allow separation of the second part from the first part when a proximal force is exerted upon this second part while the first part stays fixed, so that re-filling of the medical container is impossible.

The device of the invention may further include some or all of the features listed below.

In an embodiment, the first part and the second part are attached to each other by intereference fit, snap-fit or a clip.

In an embodiment, the proximal end of the first part includes orthoradial abutment surfaces configured for orthoradially abutting against orthoradial abutment surfaces of the second part.

In an embodiment, the second part has an opened distal end leading to an inner cavity configured for receiving an attachment member of the first part, and the opened distal end defines a distal abutment surface configured to axially abut against the first part.

In an embodiment, the plunger rod includes an axial clearance between a proximal face of the attachment member and a bottom of the inner cavity.

In an embodiment, the attachment member includes axial ribs configured for engaging axial grooves of the second part.

In an embodiment, the second part has a first portion including the axial grooves and a second portion proximally located with respect to the first portion, the first portion and the second portion being separated by a distal shoulder, the first portion having an inner diameter D3 and/or D5 greater than an inner diameter D6 of the second portion.

In an embodiment, the plunger rod has an axial clearance between a proximal end of the axial ribs and the distal shoulder.

In an embodiment, the second part has guiding members configured for guiding the axial ribs in the axial grooves when the first part and the second part are asembled to each other.

Another aspect of the invention is a medical container having a barrel defining a reservoir containing a medical product, a stopper arranged within the barrel, and the aforementioned described plunger rod placed within the barrel to push the stopper in the distal direction.

In an embodiment, the first part of the plunger rod is configured to be inacessible to a user when injection is complete.

In an embodiment, the first part of the plunger rod is configured to be fully contained within the barrel when injection is completed.

In an embodiment, the medical container is a prefilled syringe.

The invention and the advantages arising therefrom will clearly emerge from the detailed description that is given below with reference to the appended drawings as follows :
- Figure 1 is an exploded view of a plunger rod and medical container according to an embodiment of the invention,
- Figure 2A is a perspective view of a first part of a plunger rod according to an embodiment of the invention,
- Figure 2B is a perspective cross-section view of a first part of a plunger rod according to an embodiment of the invention,
- Figures 3A and 3B are perspective views of a second part of a plunger rod according to an embodiment of the invention,
- Figure 4 is a cross-section view of a plunger rod according to an embodiment of the invention,
- Figure 5A is a perspective view of a plunger rod according to an embodiment of the invention,
- Figure 5B is a detail of Figure 5A, the second part of the plunger rod being illustrated by transparency,
- Figure 5C is a cross-section view of a plunger rod according to an embodiment of the invention,
- Figures 6A to 6C illustrate use of the plunger rod and medical container according to the invention during an injection operation.

The different features of the embodiments can be used in combination with and used with other embodiments as long as the combined parts are not inconsistent with or interfere with the operation of the device and assembly. This invention is not limited in its application to the details of construction and the arrangement of components set forth in the following description or illustrated in the drawings. The embodiments herein are capable of being modified, practiced or carried out in various ways. The phraseology and terminology used herein is for the purpose of description and should not be regarded as limiting. The use of "including," "comprising," or "having" and variations thereof herein is to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not limited to physical or mechanical connections or couplings. In contrast, when an element is referred to as being "directly connected" or "directly coupled" to another element, there are no intervening elements present. Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein. Further, terms such as distal, proximal, up, down, bottom, and top are relative, and are to aid illustration, but are not limiting. Relative terms such as "below" or "above" or "upper" or "lower" or "horizontal" or "vertical" may be used herein to describe a relationship of one element to another element as illustrated in the Figures. It will be understood that these terms and those discussed above are intended to encompass different orientations of the device in addition to the orientation depicted in the Figures. The embodiments are not intended to be mutually exclusive so that the features of one embodiment can be combined with other embodiments as long as they do not contradict each other. Terms of degree, such as "substantially", "about" and "approximately" are understood by those skilled in the art to refer to reasonable ranges around and including the given value and ranges outside the given value, for example, general tolerances associated with manufacturing, assembly, and use of the embodiments. The term "substantially" when referring to a structure or characteristic includes the characteristic that is mostly or entirely present in the structure. As used herein, the singular forms "a," "an," and "the" are intended to include the plural forms as well, unless the context clearly indicates otherwise. As used herein, the term "and/or" includes any and all combinations of one or more of the associated listed items. It will be understood that, although the terms first, second, etc., may be used herein to describe various elements, these elements should not be limited by these terms. These terms are only used to distinguish one element from another. For example, a first element could be termed a second element, and, similarly, a second element could be termed a first element without departing from the scope of the present disclosure. For simplification, the parts or elements of one embodiment which are found identically or similarly in the other embodiment will be identified using the same numerical references and will not be described again.

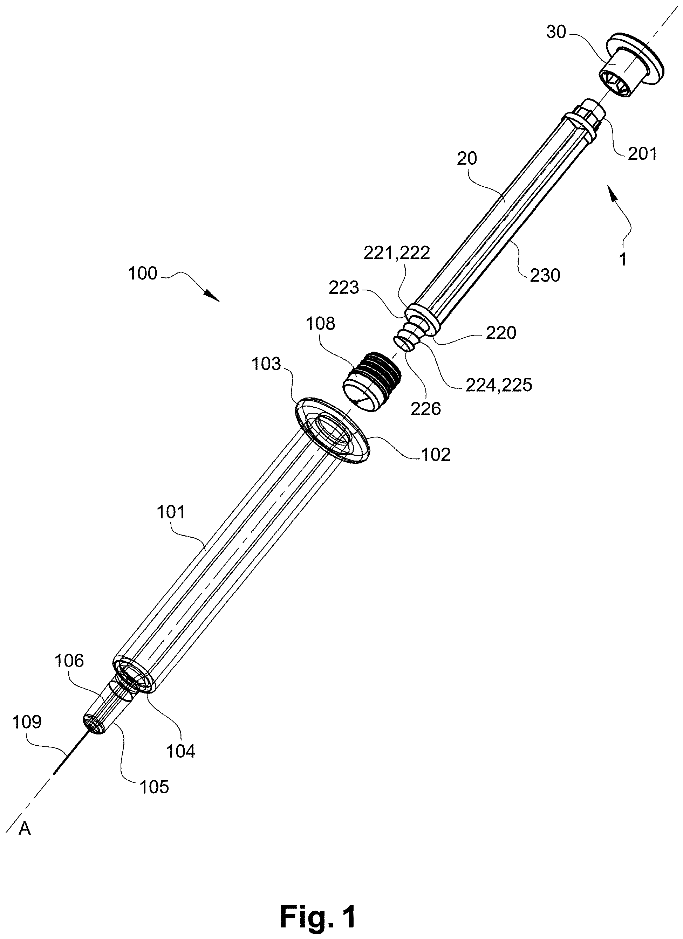

With reference to Figure 1 is shown a medical container 100 and a plunger rod 1 according to an embodiment of the invention. The medical container 100 includes a tubular barrel 101, a stopper 108, the plunger rod 1, and may also include an injection needle 109.

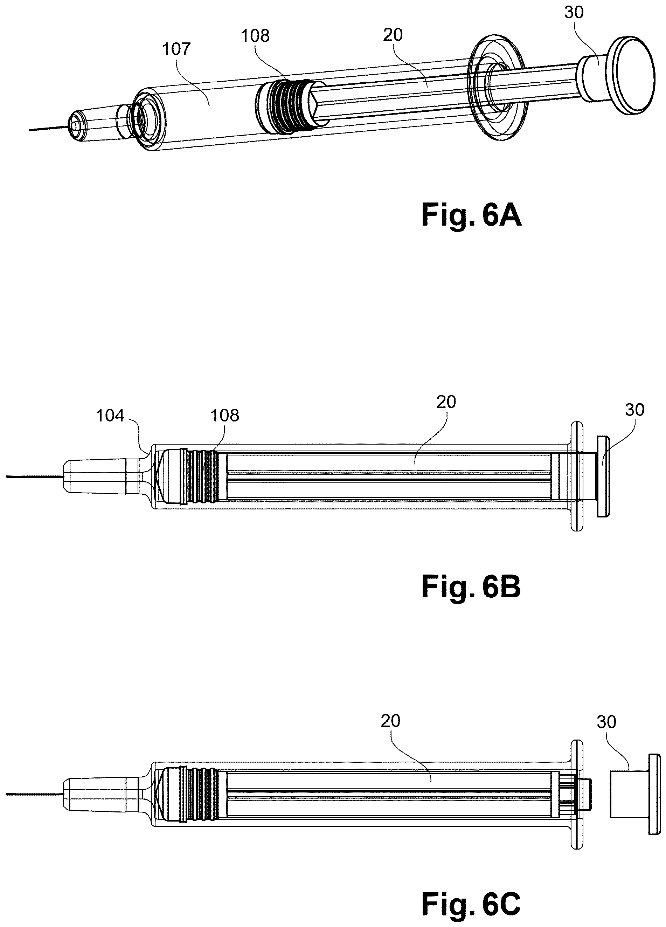

The barrel 101 is in the form of a cylindrical tube extending along a longitudinal axis A. The barrel 101 includes a proximal end 102 which may be provided with a radial flange 103, and distal end 104 which may be in the form of an elongated tip 105. The barrel 101 defines a reservoir for containing a predetermined volume of a medical product 107, Figure 6A. The proximal end 102 of the barrel 101 is opened to allow insertion of the stopper 108 and of the plunger rod 1 within the barrel 101. The elongated tip 105 defines an axial passageway 106 establishing a fluid communication between the reservoir and the injection needle 109 such that the medical product 107 is expelled from the reservoir through the elongated tip 105 and the injection needle 109 towards an injection site under the pressure of the stopper 108 being moved in the distal direction by the plunger rod 1. The barrel 101 may be made of a single piece, for instance of a glass or a plastic material.

The plunger rod 1 is made of two distinct parts 20, 30 that are configured to be removably attached to each other. The two parts 20, 30 of the plunger rod 1 are configured to remain attached to each other during the injection process, but to get detached from one another should a user pulls the plunger in the proximal direction, for example when trying to refill the syringe whereas an injection has already been performed.

The two-part plunger rod 1 thus includes a first (distal) part 20 and a second (proximal) part 30, proximally located with respect to the first part 20. The first part 20 and the second part 30 may be secured to one another by securing means configured to axially and rotationally secure the first part 20 and the second part 30 as long as the user does not exert a proximal pulling force higher than a predetermined threshold on the plunger rod 1. Preferably, the securing means are friction-fit means, so that the first part 20 and the second part 30 are attached by interference fit, although embodiments are not limited thereto. Alternatively, the first part 20 and the second part 30 may be attached to each other by a breakable member (not shown) configured to allow pushing the plunger rod 1 in the distal direction but to prevent pulling of the plunger rod 1 in the opposite direction. In any case, the securing means are designed to allow pushing of the plunger rod 1 in the distal direction to perform injection and to prevent pulling of the plunger rod 1 in the opposite direction by getting the second part 30 detached from the first part 20, thereby preventing refilling of the syringe. The securing means are thus configured such that the required proximal pulling force to detach the second part 30 from the first part 20 is lower than the required proximal pulling force for moving the stopper 108 back in the proximal direction after injection.

The first part 20 may be in the form of an axial rod which straightly extends along the central longitudinal axis A. The axial rod 20 is arranged within the barrel 101 to push the stopper 108 in the distal direction. The axial rod 20 and the stopper 108 may be fixedly attached to one another.

The axial rod 20 is movable between an initial (proximal-most) position, Figure 6A, and an end (distal-most) position, Figure 6B, distally located with respect to the initial position. The axial movement of the axial rod 20 from the initial position to the end position is caused by the user distally pushing the plunger rod 1. In the initial position, the axial rod 20 may mostly extend outside the barrel 101; in the end position, the axial rod 20 mostly or entirely extends within the barrel 101.

In the end position, a proximal end 201 of the axial rod 20 is preferably located within the barrel 101 or at the opened proximal end 102 of the barrel 101 so that the user can not pull the axial rod 20 back in the proximal direction. That is, in the end position, as shown in Figure 6C, the first part 20 preferably does not extend outside the barrel 101 but rather is fully contained within the barrel 101. If however the proximal end 201 of the axial rod 20 slightly extends outside the barrel 101, the proximal end 201 may be configured, in terms of shape or length for example, such that the user cannot grasp this proximal end 201 of the axial rod 20 or cannot pull it effectively enough to overcome the friction forces that maintain the stopper 108 together with the axial rod 20 within the barrel 101. For instance, the length of the portion of the axial rod 20 that proximally extends outside the barrel 101 in the end position may be less than one half of the axial length of the attachment member 205, or less than one half of the second portion 208 of the attachment member 205.

The axial rod 20 may be made of a single piece. The axial rod 20 may be made of a plastic material such as, for instance, polystyrene, although embodiments are not limited thereto.

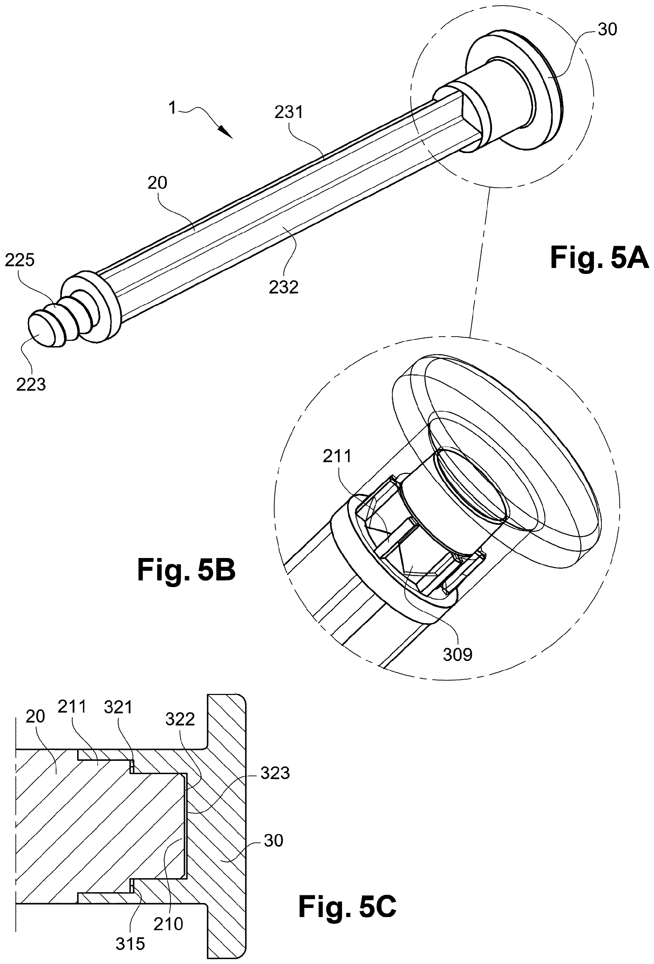

With reference to Figure 5A, the axial rod 20 includes a distal end 220, a proximal end 201, and an elongated body 230 extending from the distal end 220 to the proximal end 201.

The distal end 220 may include a disc-shaped flange 221, including a peripheral edge 222, which helps center the axial rod 20 within the barrel 101 and for guiding possible rotation of the axial rod 20 with respect to the barrel 101. The dis-shaped flange 221 of the distal end 220 may further include a distal surface 223 arranged on a distal side of this flange 221 to axially abut against the stopper 108. The distal end 220 may further include a pin 224, which distally protrudes from the flange 221 of the distal end 220 for engaging the stopper 108. The pin 224 is configured for fixedly securing the axial rod 20 to the stopper 108. Therefore, the pin 224 fits into a complementarily shaped recess (not shown) of the stopper 108, and may include attachment means, such as threads 225, in order to secure the axial rod 20 to the stopper 108. The pin 224 may include a distal pushing surface 226 to axially abut against the stopper 108 and push the stopper 108 in the distal direction.

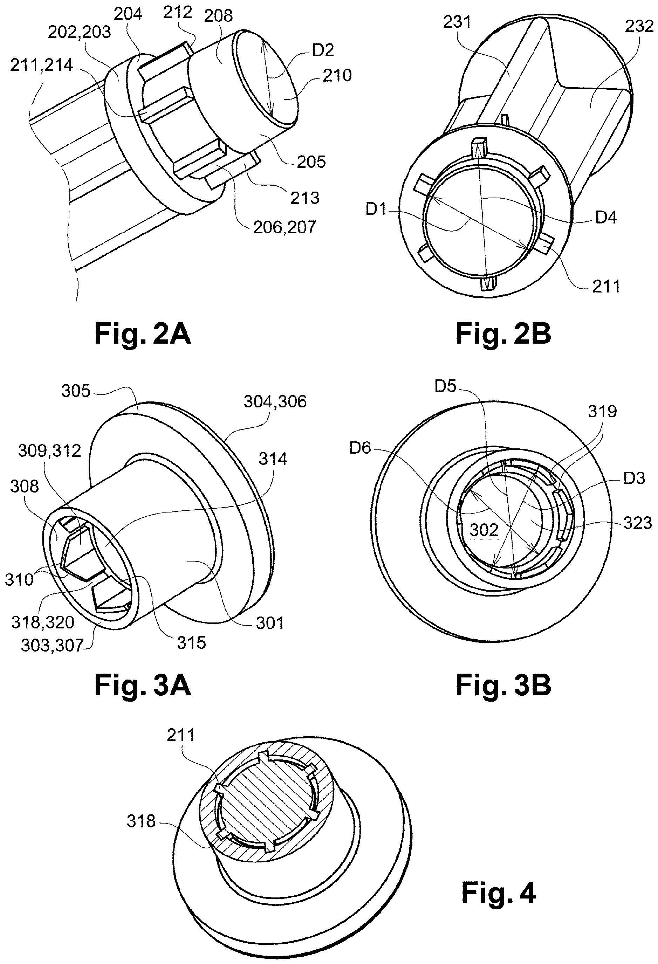

With reference to Figures 2A-2B and 5B, the proximal end 201 may include a disc-shaped flange 202 having a peripheral edge 203 forming a centering and guiding surface, a proximal stop 204 for axially abutting against the second part 30 of the plunger rod 1, an attachment member 205 for removably attaching the second part 30 to the proximal of the axial rod 20. The peripheral edge 203 is configured for radially abutting against an inner surface of the lateral wall of the barrel 101 to help guide and center the plunger rod 1 inside the barrel 101. The proximal stop 204 may be defined at a proximal side of the disc-shaped flange 202 to stop insertion of the attachment member 205 in the second part 30 of the plunger rod 1. The attachment member 205 may be in the form of an axial lug which proximally protudes from the disc-shaped flange 202. The axial lug may have a cylindrical shape.

The attachment member 205 is designed to engage a complementarily shaped recess 302 of the second part 30 of the plunger rod 1 to axially fix the second part 30 to the first part 20 of the plunger rod 1 while allowing separation of the second part 30 from the first part 20 should a user pulls the second part 30 back in the proximal direction after injection. The attachment member 205 has a first portion 206 having a first diameter D1, and a second portion 208, proximally located with respect to the first portion 206, having a second diameter D2 which may be equal to or lower than the first diameter D1. In the illustrated embodiment, the first portion 206 and the second portion 208 have the same diameter, so that D1 is equal to D2. The first diameter D1 may be equal to or preferably greater than an inner diameter D3 of the recess 302 of the second part 30, so that the second part 30 is fixed to the first part 20 of the plunger rod 1 by interference fit. That is, a lateral surface of the first portion 206 defines a friction-fit surface 207 configured to secure the second part 30 to the first part 20 of the plunger rod 1. If the first portion 206 and the second portion 208 have different diameters, they may be separated by a shoulder which may define a proximal abutment surface which may or not axially abut against the second part 30 of the plunger rod 1.

The attachment member 205 has a proximal face 210 which may or may not axially abut against a bottom 323 of the recess 302 of the second part 30 of the plunger rod 1. The attachment member 205 further includes one or more additional securing features 211 such as one more axial ribs arranged along the first portion 206 of the attachment member 205 to engage complementarily shaped securing features 318 of the second part 30, such as axial grooves. The axial ribs 211 may extend from the disc-shaped flange 202 to the second portion 208 of the attachment member 205. That is, the axial ribs 211 may extend all along the first portion of the attachment member 205, and the second portion 208 begins at the proximal end of the axial ribs 211. The axial ribs 211 may regularly distributed in a circumferential direction around the first portion 206.

The axial ribs 211 include some or all of a proximal abutment surface 212 which may be defined at a proximal end of the axial ribs 211 to axially abut against the second part 30 of the plunger rod 1, and orthoradial abutment surfaces 213 configured for orthoradially abutting against the second part 30 to rotationally fix the second part 30 with respect to the first part 20 of the plunger rod 1.

The axial ribs 211 may include an outer edge 214 forming a friction-fit surface configured for axially securing the second part 30 to the first part 20 of the plunger rod 1. As illustrated in Figure 2B, the outer edges 214 may define a diameter D4 which may be equal to or preferably greater than an inner diameter D5, Figure 3B, of the second part 30.

The elongated body 230 includes a guiding feature 231 for guiding and centering the axial rod 20 within the barrel 101, and may include one or more stiffeners 232 for rigidifying the axial rod 20 and preventing deformation. The guiding feature 231 may be delimited by an outer edge of the stiffeners 232 for radially abutting against an inner surface of the lateral wall of the barrel 101. The stiffeners 232 may be regularly distributed in a circumferential direction. The stiffeners 232 extend from the disc-shaped flange 221 of the distal end 220 of the axial rod 20 to the disc-shaped flange 202 of the proximal end 201 of the plunger rod 1.

With reference to Figures 3A-3B, the second part 30 of the plunger rod 1 is in the form of a cylindrical ring 301 defining an inner cavity 302 for receiving the attachment member 205 of the first part 20. The second part 30 is configured to help the user push the first part 20 in the distal direction during injection, and to detach from the first part 20 if pulled in the proximal direction. To that end, the second part 30 is removably attached to the first part 20. When attached, the second part 30 is axially and rotationally fixed with respect to the first part 20. The second part 30 may be made of a single piece. The second part 30 may be made of a plastic material such as polystyrene, although embodiments are not limited thereto. The first part 20 and the second part 30 may be made of the same material.

The second part 30 includes some or all of an opened distal end 303 leading to the inner cavity 302, a closed proximal end 304, a flange 305, a distal abutment surface 307, a first portion 308, a second portion 314, a guiding member 309, and one or more securing features 318.

The opened distal end 303 is configured for receiving the attachment member 205 of the first part 20. The flange 305 is arranged at the proximal end 304 of the second part 30 and closes the inner cavity 302. The flange 305 defines a pushing surface 306 configured to allow a user to push the second part 30, and thus the whole plunger rod 1, in the distal direction to expel the medical product 107 contained within the barrel 101. The flange 305 may be disc-shaped. The distal abutment surface 307 may be arranged at the distal end 303 of the second part 30 to axially abut against the first part 20 of the plunger rod 1 so that the force exerted by the user on the second part 30 is transmitted to the first part 20.

The first portion 308 is configured for accommodating the first portion 206 of the first part 20, and the second portion 314 is configured for accommodating the second portion 208 of the first part 20. The first portion 308 and the second portion 314 may be separated by a shoulder 315 which may define a distal abutment surface which may or not be configured for axially abutting against the first part 20 of the plunger rod 1, specifically against the proximal end of the axial ribs 211. As illustrated in Figure 5C, an axial clearance 321 may otherwise be provided between the proximal end of the axial ribs 211 and the shoulder 315 of the second part 30. An axial clearance 322 may also be provided between the proximal face of the attachment member 205 and a bottom 323 of the inner cavity 302.

With reference to Figures 3A and 5B, the first portion includes one or more guiding members 309, in the form inner protrusions which may be regularly distributed around the central longitudinal axis A and which may define the inner diameter D3 equal to or lower than the diameter D1 of the first part 20. The guiding members 309 accordingly include a friction-fit surface 312 for securing the second part 30 to the first part 20 of the plunger rod 1. The guiding members 309 may have guiding surfaces 310 arranged at a distal end of these guiding members 309 for easing engagement of the securing features 211 of the first and second parts 20, 30, i.e. for easing insertion of the axial ribs 211 in the axial grooves 318.

The securing features 318 of the second part 30 may indeed be in the form of axial grooves configured for complementarily receiving the axial ribs 211 of the first part 20. The axial grooves 318 are delimited between adjacent guiding members 309. The axial grooves 318 define the inner diameter D5 equal to or lower than the diamter D4 of the first part 20. The axial grooves 318 accordingly include a friction-fit surface 320 for securing the second part 30 to the first part 20 of the plunger rod 1. The axial grooves 318 have orthoradial abutment surfaces 319 for orthoradially abutting against the sides of the axial ribs 211 such that the first part 20 and the second part 30 cannot rotate relative to each other.

The friction-fit surfaces 207, 214, 312, 320 and the securing features 211, 318 form the securing means that fix the second part 30 to the first part 20 of the plunger rod 1.

The invention also relates to a medical container 100, such as a prefilled syringe, including the above-described plunger rod 1. The plunger rod 1 and medical container 100 of the invention can thus prevent re-use of the syringe should a user attempts to refill the syringe after injection.

With reference to Figures 6A-6C is described the operation of the plunger rod 1 and medical container 100 according to an embodiment of the invention.

With reference to Figure 6A, the plunger rod 1 is in the initial position. The prefilled syringe contains the medical product 107 to be expelled. The first part 20 and the second part 30 of the plunger rod 1 are fixed to one another.

The user pushes against the second part 30 of the plunger rod 1 in the distal direction, so that the plunger rod 1 moves in the distal direction within the barrel 101. The stopper 108 is thus pushed towards the distal end of the barrel 101. As a result, the medical product 107 contained in the barrel 101 is expelled via the injection needle 109 into an injection site.

With reference to Figure 6B, the plunger rod 1 is in the end position. The stopper 108 abuts against the distal end 104 of the reservoir formed by the barrel 101. The injection is complete. Only the second part 30 of the plunger rod 1 still extends outside the barrel 101. The friction force between the stopper 108 and the barrel 101 is higher than the attachment force between the second part 30 and the first part 20 of the plunger rod 1. Thus, if the user attempts to refill the barrel 101 by pulling the plunger rod 1 back in the proximal direction, the second part 30 gets detached from the first part 20 (Figure 6C). The first part 20 does not extend beyond the proximal end 102 of the barrel 101, so that the first part 20 is unattainable. The first part 20 of the plunger rod 1 stays stuck in the barrel 101. The syringe cannot be reused.

It is readily understandable from the above description that the plunger rod 1 and the medical container 100 of the invention prevent re-use of a syringe after injection. It is to be understood that the present invention is not limited to the embodiments described above and illustrated in the drawings; rather, the skilled person will recognize that many changes and modifications may be made within the scope of the appended claims.

## Claims

1. Plunger rod (1) configured for pushing a stopper (108) to expel a medical product (107) contained in a medical container (100), the plunger rod (1) including
a first part (20) having a proximal end (201) and a distal end (220) configured to push the stopper (108),
a second part (30) removably attached to the proximal end (201) of the first part (20), the second part (30) including a pushing surface (306) for allowing a user to push the second part (30) together with the first part (20) in the distal direction to perform injection, wherein the second part (30) is configured to detach from the first part (20) if pulled in the proximal direction after injection.

2. Plunger rod (1) according to the preceding claim, wherein the first part (20) and the second part (30) are attached to each other by intereference fit, snap-fit or a clip.

3. Plunger rod (1) according to any of the preceding claims, wherein the proximal end (201) of the first part (20) includes orthoradial abutment surfaces (213) configured for orthoradially abutting against orthoradial abutment surfaces (319) of the second part (30).

4. Plunger rod (1) according to any of the preceding claims, wherein the second part (30) has an opened distal end (303) leading to an inner cavity (302) configured for receiving an attachment member (205) of the first part (20), and the opened distal end (303) defines a distal abutment surface (307) configured to axially abut against the first part (20).

5. Plunger rod (1) according to the preceding claim, wherein the plunger rod (1) includes an axial clearance (321) between a proximal face of the attachment member (205) and a bottom (323) of the inner cavity (302).

6. Plunger rod (1) according to any of the preceding claims 4 or 5, wherein the attachment member (205) includes axial ribs (211) configured for engaging axial grooves (318) of the second part (30).

7. Plunger rod (1) according to the preceding claim, wherein the second part (30) has a first portion (308) including the axial grooves (318) and a second portion (314) proximally located with respect to the first portion (308), the first portion (308) and the second portion (314) being separated by a distal shoulder (315), the first portion (308) having an inner diameter D3 and/or D5 greater than an inner diameter D6 of the second portion (314).

8. Plunger rod (1) according to any of the preceding claims 6 or 7, wherein the plunger rod (1) has an axial clearance (322) between a proximal end of the axial ribs (211) and the distal shoulder (315).

9. Plunger rod (1) according to any of the preceding claims 6-8, wherein the second part (30) has guiding members (309) configured for guiding the axial ribs (211) in the axial grooves (318) when the first part (20) and the second part (30) are asembled to each other.

10. Medical container (100) having a barrel (101) defining a reservoir containing a medical product (107), a stopper (108) arranged within the barrel (101), and a plunger rod (1) according to any of claims 1-9 placed within the barrel (101) to push the stopper (108) in the distal direction.

11. Medical container (100) according to the preceding claim, wherein the first part (20) of the plunger rod (1) is configured to be inacessible to a user when injection is complete.

12. Medical container (100) according to claim 10 or 11, wherein the medical container (100) is a prefilled syringe.
